# EUROPÄISCHE PATENTSCHRIFT

(11) **EP 2 953 692 B1**
(45) Veröffentlichungstag und Bekanntmachung des Hinweises auf die Patenterteilung: **18.10.2017**
(21) Anmeldenummer: 14714929.8
(22) Anmeldetag: 05.02.2014
(51) Int. Cl.: A63B 23/18, A61M 16/00

(54) **VORRICHTUNG UND VERFAHREN ZUR DURCHFÜHRUNG EINES ATEMTRAININGS**
DEVICE AND METHOD FOR PERFORMING RESPIRATORY TRAINING
DISPOSITIF ET PROCÉDÉ PERMETTANT DE METTRE EN OEUVRE UN ENTRAÎNEMENT RESPIRATOIRE

(30) Priorität: 05.02.2013 DE 102013001913
(43) Veröffentlichungstag der Anmeldung: 16.12.2015
(73) Patentinhaber: Rheinisch-Westfälische Technische Hochschule Aachen (RWTH), 52062 Aachen (DE)
(72) Erfinder: KASHEFI-KHORASANI, Ahmad, Ali, 52074 Aachen (DE); DAUTZENBERG, Rainer, 52072 Aachen (DE)
(74) Vertreter: Cohausz Hannig Borkowski Wißgott
(86) Internationale Anmeldenummer: PCT/EP2014/000305
(87) Internationale Veröffentlichungsnummer: WO 2014/121925

(56) Entgegenhaltungen:
- WO-A2-2008/024375
- CA-A1- 1 164 304
- US-A1- 2007 221 225
- US-A1- 2008 196 726
- US-A1- 2011 212 811
- US-A1- 2012 272 956

## Beschreibung

Die Erfindung betrifft eine Vorrichtung zur Durchführung eines Atemtrainings, insbesondere eines Höhentrainings, umfassend eine Mündungsöffnung, durch die hindurch von einer Person Atemgas in ein Volumen der Vorrichtung ausatembar oder aus dem Volumen der Vorrichtung einatembar ist, wobei dieses Volumen zumindest zum Teil durch einen in seinem Behältervolumen einstellbaren, mit der Mündungsöffnung verbundenen Behälter gebildet ist.

Die Erfindung betrifft weiterhin auch ein Verfahren zur Durchführung eines Atemtrainings, insbesondere eines Höhentrainigs, wobei eine Person durch eine Mündungsöffnung in eine Trainingsvorrichtung ausatmet und aus dieser Trainingsvorrichtung einatmet, insbesondere wobei zur Durchführung des Verfahrens eine vorgenannte gattungsgemäße Vorrichtung zum Einsatz kommen kann. Eine solche Vorrichtung und Verfahren wird beispielweise im Dokument CA1164304A beschrieben.

Im Stand der Technik ist es zum Beispiel bei Sportlern bekannt, dass diese ein Atemtraining als sogenantes Höhenttraining durchführen unter reduziertem Sauerstoffpartialdruck, was ein Training in einer größeren Höhenlage simuliert, um so die sportliche Leistungsfähigkeit in niedrigeren Höhenlagen zu vergrößern. Positive Effekte beruhen hier im Wesentlichen auf einer Anpassungsreaktion des Körpers unter Hypoxiebedingungen, so dass ein solches unter reduziertem O₂-Partialdruck durchgeführtes Training die Leistungsfähigkeit physisch wie auch psychisch positiv beeinflussen kann.

Die Simulation der Atmung in einer großen Höhe wird hierbei grundsätzlich erzielt durch Einstellung eines verringerten O₂-Partialdruckes in dem einzuatmenden Atemgas gegenüber üblichen Höhenlagen wie beispielsweise auf Meeresniveau.

Statt einem tatsächlichen Training in der entsprechenden Höhe, kann dies mit einer gattungsgemäßen Vorrichtung erzielt werden, bei welcher eine trainierende Person Atemgas in ein Volumen ausatmet und aus diesem Volumen einatmet, so dass sich das eingeatmete Inspirationsgas zumindest zum Teil aus dem ausgeatmeten Atemgas zusammensetzt und demnach durch den Sauerstoffverbrauch der atmenden Person der O₂-Partialdruck abgesenkt wird. Es findet demnach im Stand der Technik quasi eine Pendelatmung statt, bei welcher die atmende Person immer einen Teil des ausgeatmeten Atemgases (Exspirationsgas) wieder einatmet.

Vorliegend wird von Atemgas statt Atemluft gesprochen, da der Begriff "Luft" eine feste bestimmte Gaszusammensetzung suggeriert, diese sich jedoch gerade mit der Vorrichtung gegenüber der Zusammensetzung normaler Luft ändert, insbesondere hinsichtlich der Partialdrücke von Sauerstoff und Kohlendioxid.

Die im Stand der Technik bekannten Vorrichtungen stellen hierfür ein Volumen bereit, das zumindest zum Teil durch einen in seinem Behältervolumen einstellbaren Behälter gegeben ist, der an eine Mündungsöffnung, beispielsweise an einem in den Mund zu nehmenden Mundstück oder aber auch an einer zu tragenden Maske angeordnet ist. Durch Wahl der Größe des Behältervolumens kann das gesamte Volumen einer solchen Vorrichtung beeinflusst werden und somit auch der Anteil des ausgeatmeten Atemgases (Exspirationsgas), das durch die trainierende Person wieder eingeatmet wird. Es lässt sich demnach durch die Wahl des Behältervolumens bei den Vorrichtungen des Standes der Technik der O₂-Partialdruck einstellen, unter dem die trainierende Person das Atemtraining, z.B. ein Höhentraining durchführt.

Bei dem hierdurch möglichen Atemtraining beziehungsweise einem Verfahren zur Durchführung eines solchen, insbesondere mit einer vorgenannten Vorrichtung wird bevorzugt ein solches Verfahren verstanden, welches nicht zu therapeutischen Zwecken seitens einer Person durchgeführt wird, sondern bevorzugt nur zu dem Zweck, die sportliche Leistungsfähigkeit zu erhöhen.

Die bislang im Stand der Technik bekannten Vorrichtungen beziehungsweise Verfahren zur Durchführung eines solchen Atemtrainings, wie beispielsweise eines Höhentrainings haben den Nachteil, dass zwar durch die Veränderung des Behältervolumens beziehungsweise des Gesamtvolumens einer entsprechenden Vorrichtung der Sauerstoffpartialdruck beeinflusst werden kann, insbesondere durch Vergrößerung des Volumens der Sauerstoffpartialdruck verringert werden kann.

Es ergibt sich jedoch im Zusammenhang mit dieser Änderung des Sauerpartialdruckes der Nachteil, dass gleichzeitig auch der CO₂-Partialdruck in dem Atemgas innerhalb des genannten Volumens und somit in dem einzuatmenden Atemgas (Inspirationsgas) beeinflusst wird, insbesondere derart, dass mit Vergrößerung des Behältervolumens und somit Verringerung des O₂-Partialdruckes sich eine Erhöhung des CO₂-Partialdruckes einstellt, was nachteilige Auswirkungen auf den Körper der trainierenden Person hat.

Es sind daher weiterhin Vorrichtungen bekannt geworden, bei denen der Vorgang des Einatmens durch einen Filter hindurch stattfindet, der mit einem Absorbermaterial gefüllt ist, um CO₂ aus der eingeatmeten Luft zu absorbieren. Auch dies ist jedoch problembehaftet, da gegebenenfalls feine Stäube des Absorbers in die Lunge der trainierenden Person gelangen können, mit gegebenenfalls negativen Auswirkungen oder aber auch dadurch, dass einzusetzende Filter, insbesondere auch solche, die das Eindringen von Feinstäuben verhindern, einen großen Atemwiderstand erzeugen.

Die Absoption des ausgeatmeten CO₂ ist eine zeitabhängige Größe, so dass, der CO₂-Partialdruck (CO₂-Konzentration) im Atemgas (Inspirationsgas) ebenfalls zeitabhängig ist. Sobald die Absoptionseffektivität nicht mehr 100% beträgt, steigt undefiniert die CO₂-Konzentration im Atemgas (Inspirationsgas) an.

Darüber hinaus existieren Anwendungsfälle, in denen es gewünscht ist, neben der Einstellung eines bestimmten O₂-Partialdruckes auch einen bestimmten CO₂-Partialdruck einzustellen, was mit den bisherigen gattungsgemäßen Vorrichtungen mangels einer ausdrücklichen Kontrollierbarkeit, insbesondere des CO₂-Partialdruckes nicht erzielt werden kann.

Es ist daher eine Aufgabe der Erfindung eine Vorrichtung der eingangs genannten gattungsgemäßen Art bereitzustellen, ebenso wie ein Verfahren zur Durchführung eines Atemtrainings, insbesondere eines Höhentrainings, mit denen sich die Möglichkeit erschließt, gezielt den O₂-Partialdruck und/oder den CO₂-Partialdruck in dem einzuatmenden Atemgas (Inspirationsgas) einer trainierenden Person einzustellen. Es ist demnach weitere Aufgabe der Erfindung, eine Vorrichtung und ein Verfahren zur Durchführung eines Atemtrainings bereitzustellen, mit denen ohne weitere Risiken für die trainierende Person im Wesentlichen beliebige Höhenlagen simuliert werden können.

Die Lösung dieser Aufgabe ist im Patentanspruch 1 und 12 angegeben.

Erfindungsgemäß wird diese Aufgabe mit einer Vorrichtung der eingangs genannten gattungsgemäßen Art gelöst, bei welcher weiterhin das Volumen der Vorrichtung, welches zumindest zum Teil durch einen in seinem Behältervolumen einstellbaren, mit einer Mündungsöffnung verbundenen Behälter gebildet ist, an eine Seite wenigstens einer gaspermeablen Membran angrenzt, an deren andere Seite wenigstens eine Gasaustauschkammer angeschlossen ist, so dass sich durch diese erfindungsgemäße Ausgestaltung die Möglichkeit erschließt, den CO₂- und/oder O₂-Partialdruck des Atemgases in dem Volumen der Vorrichtung durch die wenigstens eine gaspermeable Membran durch einen hierdurch stattfindenden Gasaustausch mit einem Fluid in der Gasaustauschkammer zu beeinflussen. Eine erfindungsgemäße Vorrichtung kann hierfür derart ausgestaltet sein, dass die Mündungsöffnung, durch die eine Person ein- und ausatmet, die beispielsweise als in den Mund zu nehmendes Mundstück ausgebildet ist oder aber auch die sich in einer das Gesicht abdeckenden Maske befindet, mit dem in seinem Behältervolumen einstellbaren Behälter verbunden ist, wobei sich das Gesamtvolumen der Vorrichtung neben dem einstellbaren Behältervolumen auch durch Zuleitungskanäle oder Schläuche zwischen Mündungsöffnung und Behälter ergibt, so wie durch gegebenenfalls andere, an der Vorrichtung vorgesehene Elemente, die ein inneres Volumen bereitstellen, wie beispielsweise einen Gasaustauschbereich, welcher gegenüberliegend der zuvor genannten Gasaustauschkammer auf der anderen Seite der wenigstens einen gaspermeablen Membran angeordnet ist und somit sich ebenso innerhalb des Volumens der Vorrichtung befindet bzw. dieses mitbildet.

Dabei ist es für die Funktionsfähigkeit der Vorrichtung unbeachtlich, ob, beispielsweise bezogen auf einen Ausatmungsprozess, die wenigstens eine gaspermeable Membran vor dem Behälter oder nach dem Behälter angeordnet ist, wenngleich erfindungsgemäß eine Vorrichtung bevorzugt wird, bei der die wenigstens eine gaspermeable Membran in Strömungsrichtung des ausgeatmeten Atemgases vor dem in seinem Behältervolumen einstellbaren Behälter positioniert ist.

Dies bedeutet, dass eine Person beim Ausatmen das Atemgas durch die Mündungsöffnung und durch einen verbindenden Kanal oder Schlauchbereich in einen Volumenteil der Vorrichtung atmet, in dem die wenigstens eine eingangs genannte gaspermeable Membran angeordnet ist, das Atemgas an dieser wenigstens eine gaspermeablen Membran entlangstreicht, hierbei ein Gasaustausch von Sauerstoff und/oder Kohlendioxid stattfinden kann und das ausgeatmete Atemgas sodann in den in seinem Behältervolumen einstellbaren Behälter gelangt.

Gegenüber den bislang im Stand der Technik bekannten Vorrichtungen und Verfahren wird hier vorteilhafter Weise erzielt, dass im Wesentlichen der O₂-Partialdruck und/oder CO₂-Partialdruck durch den Gasaustausch durch die wenigstens eine gaspermeable Membran hindurch beeinflusst wird und somit wunschgemäß ein Höhentraining beziehungsweise ein Training unter einem bestimmten gegenüber der Meereslage reduzierten O₂-Partialdruck durchgeführt werden kann, insbesondere unter reproduzierbaren einstellbaren Bedingungen.

Diese reproduzierbaren einstellbaren Bedingungen ergeben sich im Wesentlichen dadurch, dass der Gasaustausch beeinflussbar ist durch die Strömungsgeschwindigkeit bzw. den Volumenstrom des für den Gasaustausch vorgesehenen Fluides auf der Seite der Gasaustauschkammer, welche von dem Fluid durchströmt ist. Bei einem solchen für den Gasaustausch vorgesehenen Fluid kann es sich beispielsweise um ein Gas, jedoch grundsätzlich auch um eine Flüssigkeit handeln.

Die Stärke des Gasaustausches und somit die unmittelbare Beeinflussung der jeweiligen Partialdrücke von O₂ und CO₂ kann demnach maßgeblich durch die Strömungsgeschwindigkeit beziehungsweise den Volumenstrom des eingesetzten Fluides eingestellt werden.

Dabei kann bevorzugt eine aktive Durchströmung der Gasaustauschkammer mit dem vorgesehenen Fluid vorgenommen werden, beispielsweise mittels eines Antriebs, bevorzugt der von der Vorrichtung direkt umfasst ist, und mit dem das Fluid durch die Gasaustauschkammer gepumpt werden kann. Ein solcher Antrieb kann beispielsweise ein elektromotorischer Antrieb sein oder aber auch durch die Atemgasströmung von ein- und ausgeatmete Atemgas einer trainierenden Person betrieben sein, so dass eine externe Energiezuführung entbehrlich ist. Ein solcher Antrieb kann beispielsweise durch die sich ändernden Druckverhältnisse in dem Behälter oder auch durch eine Volumenänderung und eine sich dadurch ergebende mechanische Bewegung eines sich mit der Volumenänderung bewegenden Teiles erzielt werden.

Bei der erfindungsgemäßen Vorrichtung beziehungsweise der Durchführung des Verfahrens kann es im einfachsten Fall vorgesehen sein für den Gasaustausch eine einzige gaspermeable Membran vorzusehen, wobei zu der einen Seite dieser gaspermeablen Membran das ausgeatmete und/oder einzuatmende Atemgas strömt und an der anderen Seite dieser gaspermeablen Membran das für den Gasaustausch vorgesehene Fluid. Die Volumenstrombereitstellung des Fluides kann z.B. durch die trainierende Person durch sportliche Aktivitäten (Antreiben eines Ventilators durch Muskelkraft) bereitgestellt werden.

Sofern jedoch die für den Gasaustausch zur Verfügung stehende Fläche bei einer solchen Membran als nicht genügend groß angesehen wird, kann eine bevorzugte Weiterbildung es auch vorsehen, dass das Volumen der Vorrichtung an die jeweils äußere Oberfläche einer Vielzahl gaspermeabler Hohlfasern angrenzt, deren jeweiliges Inneres von einem für den Gasaustausch, insbesondere für den CO₂ oder O₂ Gasaustausch vorgesehenen Fluid durchströmbar ist, insbesondere wobei eine solche Vielzahl gaspermeabler Hohlfasern durch ein Hohlfaserbündel gebildet werden kann.

Der Volumenbereich einer erfindungsgemäßen Vorrichtung, in dem sich solche gaspermeable Hohlfasern befinden, kann demnach beispielsweise durch einen sogenannten Oxygenator gebildet werden, wie er zur Beatmung, zum Beispiel im Rahmen des Einsatzes von Herz-Lungen-Maschinen eingesetzt wird. Ein solcher Oxygenator ist üblicherweise in einem Gehäuse angeordnet, welches zwei durch die gaspermeablen Hohlfasern getrennte Volumenbereiche aufweist, wobei der eine Volumenbereich dem eingangs genannten inneren Volumen der erfindungsgemäßen Vorrichtung zugeordnet ist, insbesondere der, der an die äußere Oberfläche der Hohlfasern angrenzt und der andere Volumenbereich, dem für den Gasaustausch vorgesehenen Fluid zugeordnet ist.

Für den Gasaustausch geeignete gaspermeable Membrane beziehungsweise Hohlfasern können beispielsweise aus den folgenden Materialien ausgebildet sein: Polymethylpenthen, Silicon, Polypropylen.

Durch Wahl unterschiedlicher O₂/CO₂-Permeabilitätsverhältnisse der eingesetzten Membranen (Hohlfasern) können in der erfindungsgemäßen Vorrichtung unterschiedliche O₂- und CO₂-Transferraten und somit unterschiedliche O₂- und CO₂-Partialdrücke (Konzentrationen) eingestellt werden.

Eine Ausführung der Erfindung kann es auch vorsehen, dass die Vielzahl gaspermeabler Hohlfasern gebildet ist durch ein modular bestückbares Aufnahmeelement, in welches wenigstens ein Modul mit einer Vielzahl von Hohlfasern einsetzbar ist, bevorzugt in welches wenigstens zwei Module mit je einer Vielzahl von Hohlfasern einsetzbar ist.

In einem solchen Aufnahmeelement, das ein Gastauscher-Einheit ausbildet, grenzt das vorgenannte Volumen der Vorrichtung über das wenigstens eine Modul an die Gasaustauschkammer an. Durch die Modularität kann so auf einfache Weise eine gewünschte Transferrate durch Wahl eines bestimmten oder mehrerer bestimmter, auch untereinander unterschiedlicher Module von je einer Vielzahl von Hohlfasern eingestellt werden.

Hierbei können somit die O₂- und CO₂-Transporte in der Gastauscher-Einheit, deren Membranoberfläche und somit die O₂- und CO₂-Partialdrücke (Konzentrationen) im Inspirationsgas beeinflusst und eingestellt werden.

Eine Ausführung kann auch vorsehen, die Hohlfasern mit einem CO₂-Absorber, z.B. in den Wänden der Hohlfasern auszustatten. Bei einem modularen Aufbau kann z.B. ein Modul solche absorbierenden Hohlfasern aufweisen, insbesondere vollständig nur aus absorbierenden Holfasern gebildet sein. Ein solches Modul oder auch mehrerer solcher absorbierender Module können z.B. zusammen mit einem oder mehreren nicht absorbierenden Modulen in der vorgenannten Gasaustausch-Einheit zum Einsatz kommen. Es besteht so die Möglichkeit den CO₂-Partialdruck in der Vorrichtung weiter zu senken, ggfs. CO₂ sogar vollständig zu eliminieren.

Bei einer Ausführung der Vorrichtung, die einen Antrieb vorsieht, wie es zuvor beschrieben ist, kann es insbesondere bei der weiteren Ausgestaltung mit einer Vielzahl gaspermeabler Hohlfasern vorgesehen sein, dass durch einen solchen Antrieb ein aktives Pumpen des für den Gasaustausch vorgesehenen Fluides durch das Innere der Hohlfasern hindurch erzeugt wird, wie zuvor beschrieben, beispielsweise elektromotorisch oder auch betrieben durch die Atemgasströmung von ein- und ausgeatmetem Atemgas.

Auch bei der erfindungsgemäßen Vorrichtung beziehungsweise der Durchführung des Verfahrens kann es vorgesehen sein, das Behältervolumen des eingangs genannten Behälters einstellbar zu gestalten, wofür der Behälter in einer möglichen Ausführung, zum Beispiel als ein längenveränderbarer (Falten-)Balg ausgebildet werden kann oder aber auch aus wenigstens zwei Behälterteilen ausgebildet ist, die gegeneinander teleskopierbar sind. Es besteht so die Möglichkeit, das gesamte innere Volumen der erfindungsgemäßen Vorrichtung durch Änderung des Behältervolumens des eingesetzten Behälters variabel zu gestalten und anwendungsspezifisch einzustellen.

Dabei ist in Verbindung mit der erfindungsgemäßen Vorrichtung gegenüber dem Stand der Technik zu berücksichtigen, dass erfindungsgemäß die Größe des Behältervolumens zumindest im Wesentlichen, wenn nicht gar vollständig für die Einstellung der jeweiligen Partialdrücke unbeachtlich ist, da erfindungsgemäß die Einstellung der gewünschten Partialdrücke von O₂ und/oder CO₂ durch den stattfindenden Gasaustausch durch die gaspermeable Membran hindurch vorgenommen wird.

Vielmehr wird durch die Größe des Behälters und somit durch die Größe des gesamten inneren Volumens der Vorrichtung bei einem erfindungsgemäßen Verfahren zur Durchführung eines Atemtrainings, insbesondere eines Höhentrainings die Anzahl der Atmungen beziehungsweise die Zeit, nach welcher der gewünschte Partialdruck erreicht wird, eingestellt.

Das erfindungsgemäße Verfahren zur Durchführung eines Atemtrainings, wie z.B. eines Höhentrainings, insbesondere in Verbindung mit der zuvor beschriebenen Vorrichtung sieht vor, dass sich mit der Anzahl der Atmungen der O₂- und/oder CO₂-Partialdruck im Volumen der Trainingsvorrichtung asymptotisch an einen Endwert annähert, wobei die Größe des Endwertes eingestellt wird durch die Größe des Volumenstrom des zum Gasaustausch vorgesehenen Fluides durch die Gasaustauschkammer hindurch, welche die wenigstens eine vorgenannte Membran umfasst.

Dieses ist insbesondere der Fall bei erfindungsgemäßen Vorrichtungen, deren gesamtes inneres Volumen gegenüber der Umgebung abgeschlossen ist, wenngleich es erfindungsgemäß auch bei der Ausbildung der Vorrichtung vorgesehen sein kann, das innere Volumen der Vorrichtung gegenüber der äußeren Umgebung offen zu gestalten, z.B. durch eine Öffnung im Behälter so dass auch ein zusätzlicher Luftaustausch mit Umgebung stattfinden kann.

Die erfindungsgemäßen Vorrichtungen, insbesondere dann wenn das innere Volumen beziehungsweise der Behälter zur Umgebungsluft hin geschlossen ist, hat hier den Vorteil, dass auch untrainierte Personen unmittelbar mit einer solchen Vorrichtung ein Atemtraining beginnen können, und so dann die Möglichkeit besteht, durch das Behältervolumen den Verlauf der asymptotischen Annäherung an der Partialdruck-Endwert an den Untrainierten anzupassen, insbesondere so, dass dieser Endwert vergleichsweise langsam erreicht gegenüber einer kleineren Wahl des Behältervolumens beziehungsweise des Gesamtvolumens der Vorrichtung für eine trainierte Person, bei welcher der Partial-Enddruck schneller erreicht werden kann.

So erfolgt beispielsweise ein schnelleres Erreichen des Endpartialdruckes von O₂ beziehungsweise CO₂ bei einem vergleichsweise kleinen Behältervolumen beziehungsweise Gesamtvolumen der Vorrichtung und ein langsameres asymptotisches Angleichen an den Enddruck bei dem gegenüber vergleichsweise größeren inneren Volumen beziehungsweise Behältervolumen.

Bei einer erfindungsgemäßen Ausführungsform, bei welcher das innere Volumen der Vorrichtung beziehungsweise der Behälter, der maßgeblich zum inneren Volumen der Vorrichtung beiträgt, zur Umgebungsluft hin geschlossen ist, kann es zur Weiterbildung vorgesehen sein, dass ein sogenanntes Atemzugbehältnis einen Teil des Volumens bildet, insbesondere welches direkt an den einstellbaren Behälter angrenzt, insbesondere in Flussrichtung der ausgeatmeten Luft nach dem Behälter angeordnet ist, wobei dieses Atemzugbehältnis bewegliche und/oder flexible Wandbereiche aufweist, so dass das Volumen des Atemzugbehältnisses durch ein- und ausströmendes Atemgas vergrößerbar und verkleinerbar ist.

So trägt eine solche Ausführungsform dem Umstand Rechnung, dass bei einem gegenüber der Umgebungsluft hin geschlossenen inneren Volumen dieses Volumen nicht konstant sein darf, da sich ansonsten mit Ausatmen von Luft der Druck in der Vorrichtung erhöht und dem Prozess des Ausatmens einen erhöhten Widerstand entgegenbringt.

Vielmehr ist es gewünscht, dass eine trainierende Person immer mit gleichem Atemwiderstand trainieren kann, so dass zur Erzielung dieses Zweckes ein solches Atemzugbehältnis vorgesehen ist, welches sein eigenes Volumen durch die ein- und ausströmende Luft vergrößert beziehungsweise verkleinert, dadurch dass die beweglichen oder flexiblen Wandbereiche, die zum Beispiel durch eine elastische Membran oder eine bewegliche Folie ausgebildet sein können, sich durch eine sich einstellende Druck- beziehungsweise Volumenvergrößerung bewegen, wodurch die Volumenvergrößerung den Druck in der Vorrichtung auf im wesentlichen gleichen Niveau hält.

Hierbei kann eine Weiterbildung vorsehen, dass die Bewegungsweite und damit das maximale Volumen, dass ein Atemzugbehältnis einnehmen kann, begrenzbar ist, insbesondere variabel begrenzbar ist durch wenigstens ein verstellbares Anschlagelement, an welches die beweglichen oder flexiblen Wandbereiche des Atemzugbehältnisses bei maximaler Anfüllung mit Atemgas anlegbar sind. So kann es sich beispielsweise bei einem solchen Anschlagelement um einen Gehäusebereich handeln, der beispielsweise perforiert ist und in den hinein die flexiblen Wandbereiche, wie beispielsweise eine bewegliche Folie oder eine flexible Membran eintritt und sich an die perforierten Wandbereiche anlegt, die sodann eine weitere Bewegung dieser beweglichen Wandbereiche unterbindet und das maximale Volumen begrenzt.

Ein solches Atemzugbehältnis, das gemäß der vorgenannten Ausführungsform bevorzugter weise in seinem maximalen Volumen einstellbar ist, kann bei der Durchführung des Trainings bevorzugt so eingestellt werden, dass das Volumen, welches das Atemzugsbehältnis an dem Gesamtvolumen der Vorrichtung einnimmt, dem sogenannten Atemzugvolumen der trainierenden Person entspricht, somit also im Wesentlichen der Menge an Atemgas, die eine Person beim Ein- und Ausatmen bewegt und die somit zumindest im Wesentlichem dem maximalen Lungenvolumen entspricht.

Hier kann es jedoch in einer weiteren Weiterbildung vorgesehen sein, dass am Volumen, insbesondere am einstellbaren Behälter oder an dem Atemzugbehältnis wenigstens ein Ventil angeordnet ist, durch welches beim einem Überdruck gegenüber der äußeren Umgebung Atemgas aus dem Volumen zur Umgebung hin ablassbar ist und/oder wenigstens ein Ventil angeordnet ist, durch welches bei einem Unterdruck gegenüber der äußeren Umgebung Frischluft in das Volumen einsaugbar ist.

Hierdurch kann besonders dem Umstand Rechnung getragen werden, dass das maximale Volumen des Atemzugbehältnisses gegebenenfalls kleiner gewählt wird als das Atemzugvolumen der trainierenden Person, so dass in einem solchen Fall, wenn die Person mehr Volumen in die erfindungsgemäße Vorrichtung beim Ausatmen hineinbläst als das Atemzugbehältnis aufnehmen kann, dieses überschüssige ausgeatmete Atemgas (Expirationsgas) über das Ventil nach außen zur Umgebung hin abgelassen wird und im umgekehrten Fall, wenn die Person mehr Atemgas (Inspirationsgas) einatmet, als das Atemzugbehältnis an variablem Volumenanteil zur Verfügung stellt, durch den sich dann einstellenden Unterdruck Frischluft aus der Umgebung über das wenigstens eine Ventil angesaugt wird.

So wird in beiden Fällen sichergestellt, dass in keinem möglichen Fall bei den Atembewegungen einer trainierenden Person die erfindungsgemäße Vorrichtung den Atemvorgang Widerstände entgegenstellt.

Eine mögliche erfindungsgemäße Ausführungsform kann es vorsehen, dass sowohl beim Vorgang des Ausatmens als auch beim Einatmen jeweils das strömende Atemgas an der wenigstens einen gaspermeablen Membran, insbesondere an den vielen gaspermeablen Hohlfasern vorbeiströmt, so dass bei diesen beiden sich periodisch wiederholenden Atembewegungen jeweils ein Gasaustausch von O₂ und/oder CO₂ stattfinden kann.

Eine andere Ausführungsform kann es auch vorsehen, dass entweder nur beim Ausatmen oder nur beim Einatmen das bewegte Atemgas innerhalb der erfindungsgemäßen Vorrichtung an der wenigstens einen Membran zum Zwecke des Gasaustausches vorbeiströmt.

Hier kann es vorgesehen sein, dass durch wenigstens ein Einwegventil und wenigstens einen Bypass-Kanal für die jeweils andere Strömungsrichtung ein Umgehungsweg bereitgestellt wird, so dass die jeweils in der anderen Richtung strömende Luft nicht an der wenigstens einen gaspermeablen Membran vorbeigeführt wird. Insbesondere kann es hier vorgesehen sein, dass nur das beim Ausatmen in die Vorrichtung eingeblasene Atemgas (Exspirationsgas) an der wenigstens einen gaspermeablen Membran vorbeigeführt wird, jedoch durch wenigstens ein Einwegventil und eine Bypass-Leitung dafür Sorge getragen wird, dass beim Einatmen das Atemgas direkt aus dem in seinem Volumen veränderbaren Behälter und/oder dem Atemzugbehältnis unter Umgehung eines Gasaustausches der Mündungsöffnung zugeführt wird.

Erfindungsgemäße Ausführungsformen und Charakteristika des Verfahrens werden nachfolgend beschrieben.

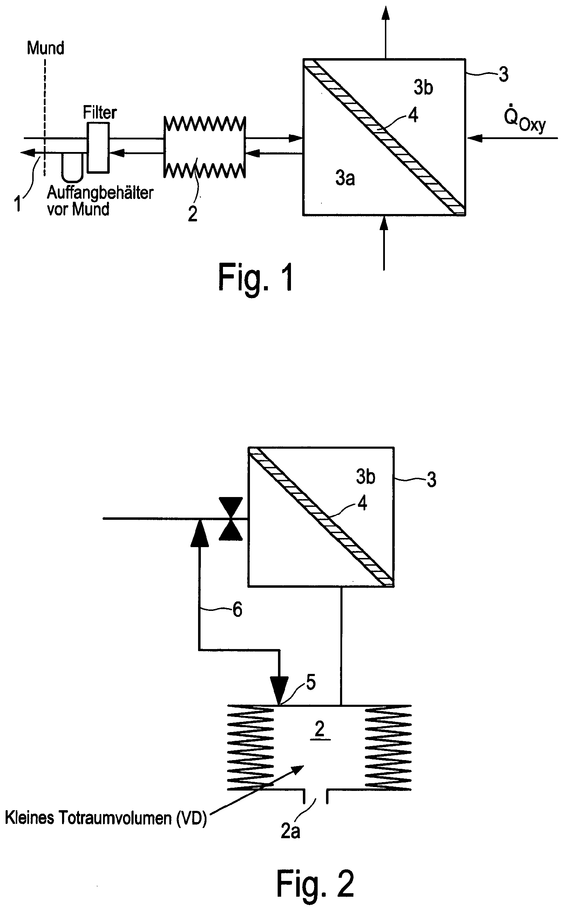

Die Figur 1 zeigt eine prinzipielle schematische Darstellung einer erfindungsgemäßen Vorrichtung beziehungsweise die Durchführung des erfindungsgemäßen Verfahrens, wobei eine hier nicht dargestellte Person durch eine nur schematisch skizzierte Mündungsöffnung 1 atmet und hierbei das Atemgas in die erfindungsgemäße Vorrichtung eingeblasen beziehungsweise aus dieser eingeatmet wird, die gemäß der hier schematisch dargestellten Abbildung einen in seinem Behältervolumen einstellbaren Behälter 2 umfasst sowie eine Gasaustauschvorrichtung 3, deren inneres Volumen in einen Volumenteil 3a und einen Volumenteil 3b durch wenigstens eine gaspermeable Membran 4 unterteilt ist, wobei der Volumenteil 3a dem gesamten inneren Volumen der erfindungsgemäßen Vorrichtung zugerechnet wird und der Volumenteil 3b der beschriebenen Gasaustauschkammer, durch die ein für den Gasaustausch vorgesehenes Fluid geführt wird.

Es wird demnach hier das ein- und ausgeatmete Atemgas an der wenigstens einen gaspermeablen Membran 4 entlanggeführt, um so einen Gasaustausch mit dem auf der anderen Seite der wenigstens einen Membran fließenden Fluid stattfinden zu lassen, so dass durch die Strömungsgeschwindigkeit beziehungsweise den Volumenstrom dieses Fluides der endgültig zu erreichende O₂- beziehungsweise CO₂-Partialdruck innerhalb der erfindungsgemäßen Vorrichtung eingestellt werden kann.

Wie bereits eingangs beschrieben, wird durch die Wahl der Größe des Volumens des in seinem Behältervolumen einstellbaren Behälters 2 Einfluss genommen auf das gesamte innere Volumen der erfindungsgemäßen Vorrichtung, welche sich somit zusammensetzt zumindest aus dem inneren Behältervolumen des Behälters 2, dem Volumen 3a der für den Gasaustausch vorgesehenen Einrichtung 3 sowie gegebenenfalls weiterer dieser Vorrichtungsteile verbindende Elemente, wie Kanäle oder Schläuche oder sonstige das Atemgas führende Bauteile der gesamten erfindungsgemäßen Vorrichtung.

Die Figur 1 zeigt, dass der Behälter 2 der in seinem Volumen einstellbar ist, zum Beispiel dadurch, dass er als Balg ausgebildet ist, in Strömungsrichtung des ausgeatmeten Atemgases (Exspirationsgas) vor der Gasaustauschvorrichtung mit den beiden Volumenanteilen 3a und 3b und der wenigstens einen gaspermeablen Membran angeordnet ist. Unter Erzielung der im Wesentlichen gleichen Funktion kann der Behälter 2 auch in Strömungsrichtung des ausgeatmeten Atemgases nach der für den Gasaustausch vorgesehenen Vorrichtung 3 angeordnet werden, wie dies als Alternative ohne Darstellung der Mündungsöffnung die weitere Figur 2 zeigt, bei welcher das Gesamtvolumen der erfindungsgemäßen Vorrichtung gegenüber der Umgebung hin offen ist, dadurch, dass der Behälter 2 eine Öffnung 2a zur Umgebung hin aufweist.

Das ausgeatmete Atemgas(Exspirationsgas) kann demnach auch zur Umgebungsluft hin entweichen, ebenso wie eine Person beim Einatmen Umgebungsluft durch die gesamte Vorrichtung einatmen kann. Die Figur 2 zeigt hierbei auch eine Ausführungsvariante, bei welcher je nach Richtung von nicht dargestellten Einwegventilen 5 einstellbar ist, ob Atemgas nur beim Ausatmen oder nur beim Einatmen über die Bypass-Leitung 6 an der wenigstens einen gaspermeablen Membran 4 vorbeigeführt werden soll, demnach immer nur in einer der beiden Atemphasen (Einatmen beziehungsweise Ausatmen) ein Gasaustausch mit dem Fluid in dem Volumenbereich 3b des Vorrichtungsteil 3 stattfindet.

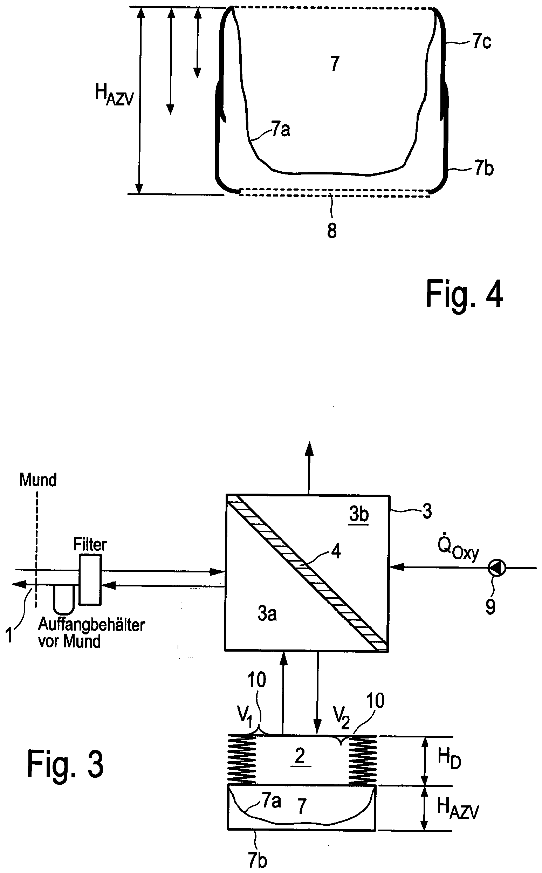

Eine wiederrum andere Ausführungsvariante der erfindungsgemäßen Vorrichtung beziehungsweise der Durchführung des Verfahrens ist in der Figur 3 dargestellt, welche gegenüber den Figuren 1 und 2 zeigt, dass zum einen der in seinem Behältervolumen variabel einstellbaren Behälter 2 in Strömungsrichtung des ausgeatmeten Atemgases (Exspirationsgas) nach der Vorrichtung 3 zum Gasaustausch angeordnet ist und weiterhin an den Behälter 2 ein Atemzugbehältnis 7 angeordnet ist, das Teil des gesamten inneren Volumens der erfindungsgemäßen Vorrichtung ist und bewegliche und/oder flexible Wandbereiche 7a aufweist, zum Beispiel dadurch, dass diese als bewegliche Folie ausgebildet sind oder als bewegliche elastische Membran, so dass das Volumen des Atemzugbehältnisses 7 durch ein- und ausströmendes Atemgas vergrößerbar und verkleinerbar ist, wobei die Figur 3 weiterhin zeigt, dass das maximale Volumen des Atemzugbehältnisses 7 begrenzt wird durch ein Anschlagelement 7b, dass durch die äußeren Gehäusewandungen des Atemzugbehältnisses 7 realisiert wird. Hierdurch wird bewirkt, dass die beweglichen Wandbereiche 7a beim Ausatmen der Volumenvergrößerung folgen, bis dass sich diese vollständig an das begrenzende Anschlagelement 7b anlegen und somit das Volumen des Atemzugbehältnisses 7 nicht weiter vergrößert werden kann.

Die Figur 4 zeigt ein Detail des Atemzugbehältnisses 7 mit dem flexiblen Wandbereichen 7 und dem Anschlagelement 7b, wobei sich aus dieser Detaildarstellung ergibt, dass das maximale Volumen dieses Atemzugbehältnisses 7 variabel eingestellt werden kann, dadurch dass das Anschlagelement 7b gegenüber einem anderen, zum Beispiel mit dem Behälter 2 festen Gehäuseteil 7c verschiebbar ist, insbesondere teleskopierbar ist. Auch hier könnte, wie beim Behälter 2, auch die Ausführung als Balg vorgesehen sein. Zumindest ein Wandbereich, der als Anschlagelement 7b dienenden Gehäusewandung ist hier mit Perforationen 8 ausgebildet, damit bei der Expansion, also mit Volumenvergrößerung, die Luft außerhalb der beweglichen und/oder flexiblen Wandbereiche 7a aus dem Gehäuse des Atemzugbehältnis 7 verdrängt werden kann und keinen Widerstand entgegenstellt.

Die Ausführung gemäß der Figur 3 ist dergestalt, dass sowohl das eingeatmete als das ausgeatmete Atemgas an der wenigstens einen gaspermeablen Membran 4 vorbeigeführt wird und demnach in beiden Atemphasen ein Gasaustausch mit dem Fluid in dem Volumen 3b stattfinden kann, wofür hier mit einem Antrieb 9 ein gewünschter Volumenstrom des für den Gasaustausch vorgesehenen Fluides wählbar ist. In diesem Fall kann der Antrieb 9 ein elektromotorischer Antrieb, insbesondere eine Pumpe sein.

Die Figur 3 zeigt weiterhin, dass insbesondere an dem Behälter 2 gegebenenfalls allerdings auch an jedem anderen das Gesamtvolumen der erfindungsgemäßen Vorrichtung begrenzenden Wandbereich Ventile 10 vorgesehen sein können, die als Einwegventile dienen und im vorliegenden Fall eingesetzt sind bei einem sich einstellenden Überdruck innerhalb der Vorrichtung beim Ausatmen das ausgeatmete Atemgas (Exspirationsgas) zur Umgebung hin zu entlassen beziehungsweise Umgebungsluft in die Vorrichtung einzuatmen, sofern beim Vorgang des Einatmens ein Unterdruck entsteht. Diese Effekte können insbesondere dann Auftreten, wenn das variabel einstellbare Volumen des Atemzugbehältnisses 7 kleiner eingestellt wird als das Atemzugvolumen der trainierenden Person, welches zum Beispiel mit deren Lungenvolumen gleich sein kann.

Wird im Gastauscher-Einheit mehr CO₂ eliminiert als O₂ transportiert, entsteht im Laufe der Anwendung ein Defizit an Volumen in der Vorrichtung. Hier kann dann gegebenfalls Luft aus der Umgebung in die Vorrichtung eingesogen werden.

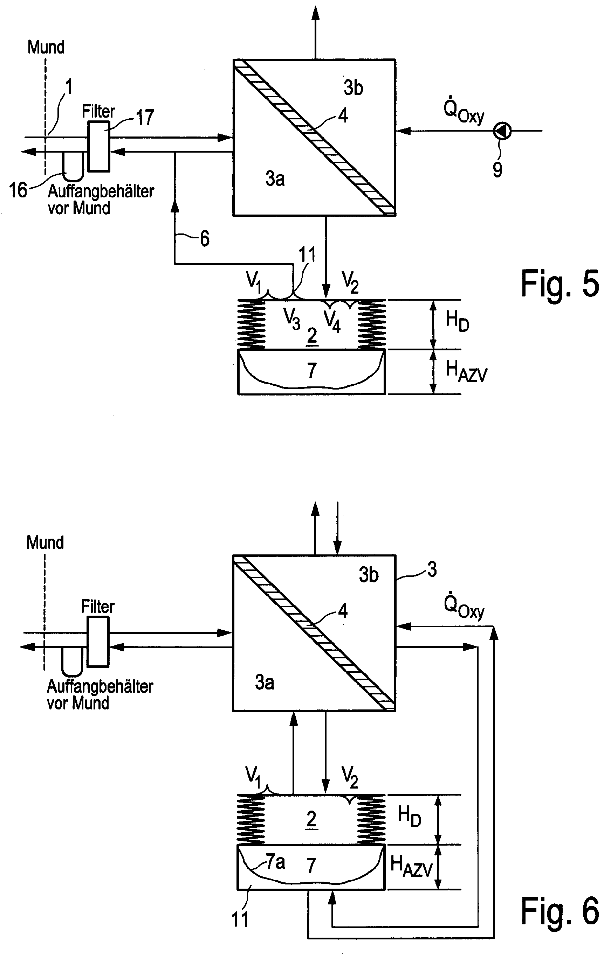

Als Alternative zur Figur 3 zeigt die Figur 5 eine Ausführungsform, bei der so wie bei der Figur 2 eine Bypass-Leitung 6 vorgesehen ist, die über ein Einwegventil 11 im vorliegenden Fall direkt am Behälter 2 angeordnet ist und bewirkt, dass beim Vorgang des Einatmens die Luft aus dem Behälter 2 und dem Atemzugbehältnis 7 unter Umgehung der gaspermeablen Membran 4 direkt der Mündungsöffnung 1 zugeführt wird. Bei der Ausführung gemäß der Figur 5 wird demnach lediglich beim Ausatmen ein Gasaustausch stattfinden, wenn das ausgeatmete Atemgas (Exspirationsgas) an der wenigstens einen gaspermeablen Membran 4 entlanggeführt wird, nicht jedoch beim Einatmen. Die Richtung der Luftführung innerhalb des Bypasses 6 kann durch entsprechende Wahl des Einwegventiles 11 auch umgekehrt werden.

Figur 6 zeigt wiederum eine andere Ausführungsform, die insbesondere mit denjenigen Ausführungen kombinierbar ist, die ein Atemzugbehältnis 7 aufweisen und bei denen das innere Volumen der gesamten erfindungsgemäßen Vorrichtung gegenüber der Umgebung hin abgeschlossen ist. Diese Ausführung verdeutlicht, dass durch die Bewegung der beweglichen Wandbereiche 7a im Atemzugbehältnis beim Aus- und Einatmen das auf der anderen Seite, das heißt außerhalb des inneren Volumens der erfindungsgemäßen Vorrichtung liegende Luftvolumen 11 verringert oder vergrößert wird. Die durch den Volumenbereich 11 verdrängte oder eingezogene Luft beziehungsweise alternativ auch eines jeglichen Fluids, kann ebenso eine Bewegung und ein Volumenstrom im Kammerteil 3b der gasaustauschenden Vorrichtung 3 hervorrufen, so dass anstelle eines elektromotorischen Antriebes der Volumenstrom eines gasaustauschenden Fluides auf der einen Seite der wenigstens einen gaspermeablen Membran 4 auch unmittelbar durch die Atembewegungen der trainierenden Person erzeugt wird.

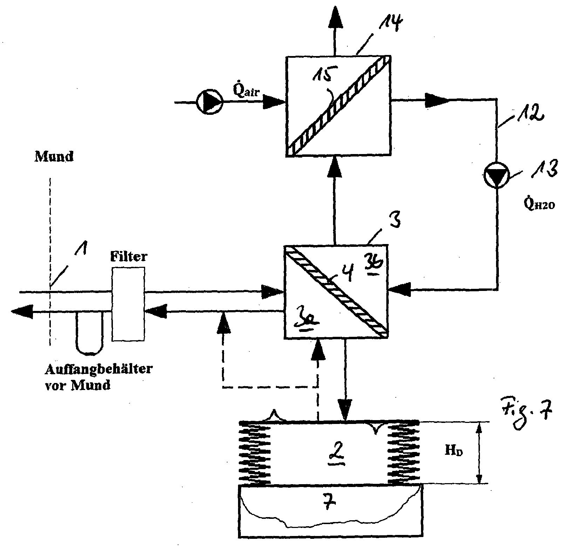

Figur 7 zeigt eine weitere Ausführungsform, die im Wesentlichen mit denen der vorherigen Ausführungen übereinstimmen kann, wobei hier als gasaustauschendes Fluid zum Zwecke der Einstellung der Partialdrücke von O₂ und CO₂ im inneren Volumen der erfindungsgemäßen Vorrichtung Wasser eingesetzt wird, welches in einem geschlossenen Kreislauf 12 gefördert wird, insbesondere mit einem elektromotorischen Antrieb 13, wobei innerhalb dieses Kreislaufes zwei gasaustauschende Vorrichtungen angeordnet sind, nämlich eine zur Einstellung des O₂ und CO₂-Partialdruckes im inneren Volumen der erfindungsgemäßen Vorrichtung und eine weitere gasaustauschende Vorrichtung 14 vorgesehen ist, ebenso mit wenigstens einer gaspermeablen Membran 15, um den Gasaustausch zwischen Wasser innerhalb des Kreislaufes 12 und einem weiteren Fluid, beispielsweise Luft, vorzunehmen.

Sämtliche Ausführungsformen der vorbeschriebenen Figuren zeigen, dass der Mündungsöffnung, die zum Beispiel in einem Mundstück oder in einer das Gesicht überdeckenden Maske angeordnet ist, zusätzlich auch noch ein Auffangbehälter 16, zum Beispiel für Speichel aus dem Mund der trainierenden Person vorgesehen sein kann oder aber auch für kondensierende Feuchtigkeit innerhalb des Atemgases sowie gegebenenfalls auch noch ein Filter, insbesondere Gas-Filter 17. Diese Elemente und ein Filter zur Anfnahme der Gasfeuchtigkeit sind für das erfindungsgemäße Verfahren beziehungsweise die Vorrichtung nicht zwingend beziehungsweise wesentlich und können bei sämtlichen dargestellten Ausführungsformen auch entfallen.

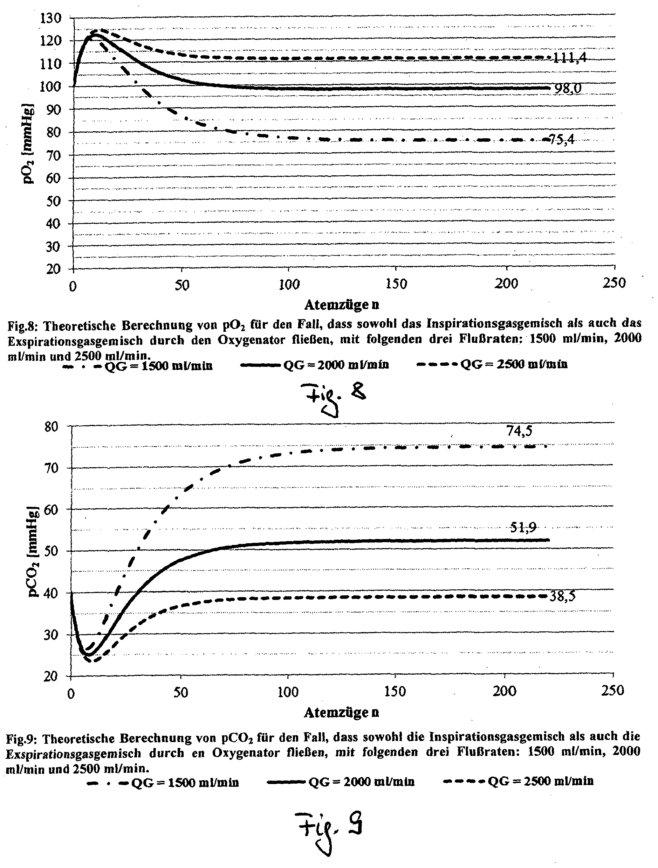

Die Figuren 8 und 9 zeigen die Entwicklung der Partialdrücke von O₂ (Figur 8) beziehungsweise CO₂ (Figur 9) bei verschieden eingestellten Flussraten des gasaustauschenden Fluides durch die gasaustauschende Kammer 3. Hier ist erkennbar, dass in Abhängigkeit des Volumenstromes der partielle Enddruck von O₂ beziehungsweise CO₂ im Inneren des Volumens der erfindungsgemäßen Vorrichtung eingestellt werden kann.

Dieser Enddruck wird jeweils mit einem asymptotischen Verlauf erreicht nach einer gewissen Anzahl von Atembewegungen n die auf der jeweiligen x-Achse der Figur aufgetragen sind. Die Figuren 8 und 9 belegen somit, dass gegenüber dem Stand der Technik reproduzierbar einstellbar durch die Flussrate des gasaustauschenden Fluides die Trainingsbedingungen für eine Person gewählt werden können, nämlich der jeweils zu erzielende Partialdruck von O₂ beziehungsweise CO₂ und somit auch die bei einem Training zu simulierende Höhe.

Dabei zeigen die Figuren 8 und 9 den typischen Verlauf des Partialdruckes von O₂ und CO₂ für den Fall, dass sowohl beim Ausatmen als auch beim Einatmen das innerhalb der Vorrichtung bewegte Atemgas an der wenigstens einen gaspermeablen Membran 4 der Vorrichtung vorbeigeführt wird.

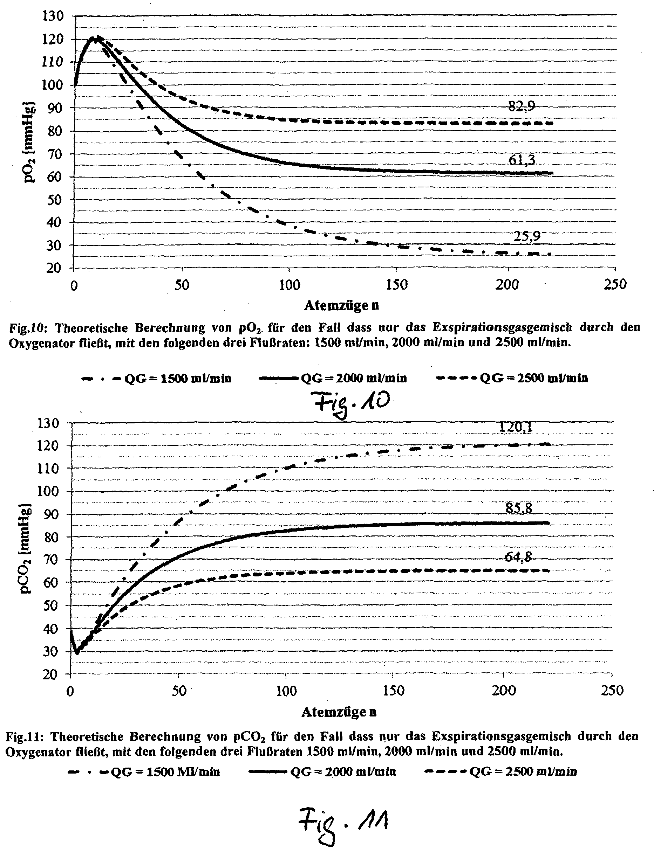

Die Figuren 10 und 11 zeigen dieselbe Situation für dieselben Flussraten des gasaustauschenden Fluides für den Fall, dass nur beim Ausatmen die in der Vorrichtung bewegte Luft einem Gasaustausch unterliegt, also an der wenigstens einen gaspermeablen Membran 4 entlanggeführt wird, hingegen das eingeatmete Atemgas (Inspirationsgas) mittels einer Bypass-Leitung und einem Einwegventil direkt der Mündungsöffnung zugeführt wird, wie dies beispielsweise die Figuren 2 und 5 visualisieren.

Hier zeigt sich, dass bei den selben Flussraten beim dem Sauerstoffpartialdruck ein geringerer Druck erzielt wird im Vergleich zum zweimaligen Gasaustausch, hingegen beim dem CO₂-Partialdruck ein höherer Druck im Vergleich zu dem zweimaligen Gasaustausch der Figuren 8 und 9. So lässt sich demnach auch dadurch Einfluss nehmen auf die jeweils zu erzielenden Partialdrücke von Sauerstoff und CO₂, in dem ausgewählt wird, ob die in der Vorrichtung bewegte Luft nur beim Einatmen, nur beim Ausatmen oder bei beiden Atembewegungen einem Gasaustausch unterliegt.

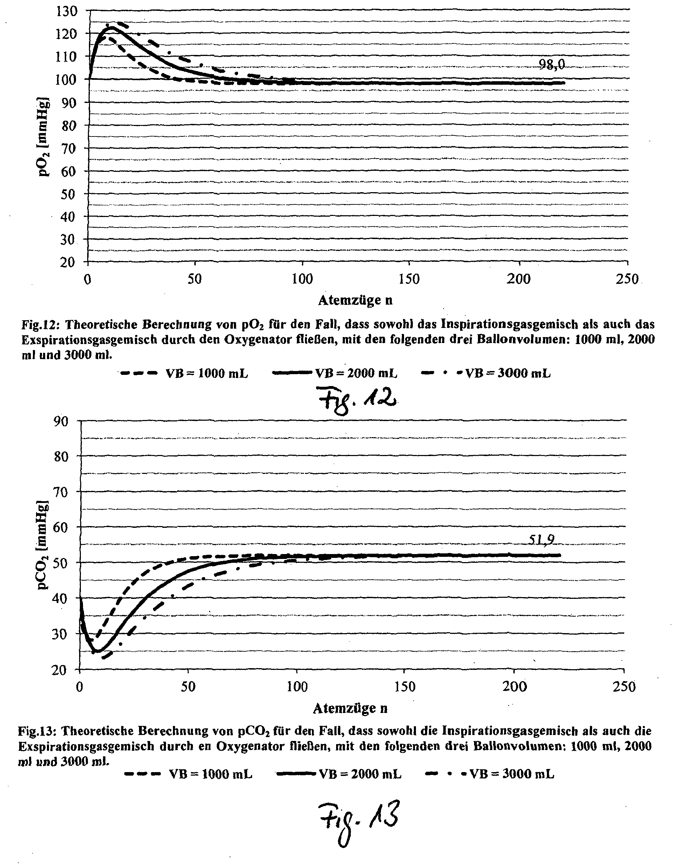

Die Figuren 12 und 13 zeigen wiederum für den Partialdruck von O₂ und CO₂ die jeweilige Entwicklung der Partialdrücke in Abhängigkeit der Anzahl der Atembewegungen und für verschiedene Größen des gesamten inneren Volumens der jeweiligen erfindungsgemäßen Vorrichtung, die hierdurch unterschiedlich eingestellte Behältervolumina der jeweils zuvor beschriebenen Behälter 2 ausgewählt werden. Es zeigt sich hier, dass der durch den Volumenstrom der am Gasaustausch teilnehmenden Fluide eingestellte jeweilige Partialdruck von O₂ und CO₂ in Abhängigkeit des Volumens der Vorrichtung beziehungsweise des Behälters unterschiedlich schnell erreicht wird.

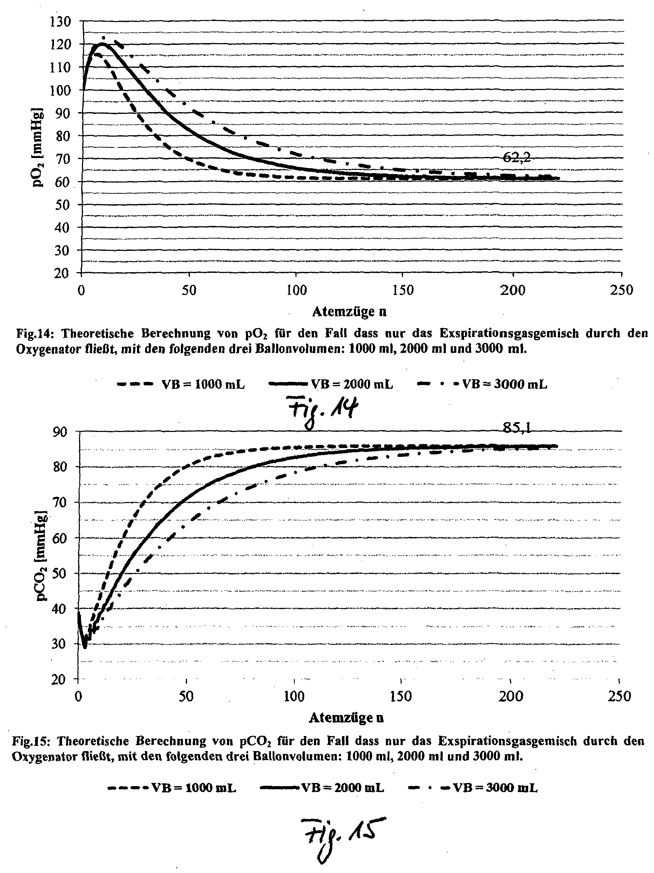

Hierbei stellen die Figuren 12 und 13 den zu den Figuren 8 und 9 zugehörigen Fall dar, wenn sowohl beim Einatmen als auch beim Ausatmen das Atemgas (Inspiration- und Exspirationsgas) an der wenigstens einen gaspermeablen Membran entlanggeführt wird. Dem gegenüber zeigen die Figuren 14 und 15 denselben Fall, wenn nur die ausgeatmete Luft einem Gasaustausch unterliegt, sowie es auch zu den Figuren 10 und 11 beschrieben wurde.

Hier sind sowohl die jeweils erreichten Grenzwerte verändert als auch die Abhängigkeit vom Volumen der Vorrichtung signifikant erkennbar.

Insbesondere erschließt sich hier bei der Durchführung des Verfahrens zum Trainieren einer Person die Möglichkeit eine untrainierte Person mit einem größeren Volumen der Vorrichtung trainieren zu lassen, damit diese den eingestellten Endwert des Sauerstoff beziehungsweise Kohlendioxid-Partialdruckes über eine längere Zeit hin erreicht, als eine trainierte Person, bei welcher das Volumen der Vorrichtung geringer gewählt werden kann.

Bezüglich der hier schematisch dargestellte Vorrichtungen der Figuren 1 bis 7 ist festzuhalten, dass lediglich zur Vereinfachung der Darstellung in der gasaustauschenden Vorrichtung 3 eine einzige gaspermeable Membran 4 visualisiert ist, ebenso wie bei der Vorrichtung 14 nur eine Membran 15, wohingegen beim praktischen Einsatz bevorzugt ein Bündel von vielen Gaspermeablen Hohlfasern vorgesehen sein kann, insbesondere deren Äußeres von dem Atemgas angeströmt ist und deren Inneres durch das gasaustauschende Fluid beaufschlagt wird.

Bei gleichen Anfangs- und Randbedingungen können bei unterschiedlichen Anwendern unterschiedliche O₂- und CO₂-Verläufe (in Abhängigkeit der Zeit) gemessen werden. Die Vorrichtung kann somit zur Beurteilung der Lunge des Anwenders verwendet werden.

## Patentansprüche

1. Vorrichtung zur Durchführung eines Höhentrainings umfassend eine Mündungsöffnung (1), durch die hindurch von einer Person Atemgas in ein Volumen (2, 3a, 7) ausatembar oder aus dem Volumen (2, 3a, 7) einatembar ist, wobei dieses Volumen (2, 3a, 7) zumindest zum Teil durch einen in seinem Behältervolumen einstellbaren, mit der Mündungsöffnung verbundenen Behälter (2) gebildet ist und wobei das Volumen (2, 3a, 7) an eine Seite wenigstens einer gaspermeablen Membran (4) angrenzt, **dadurch gekennzeichnet, dass** an der anderen Seite der wenigstens einen Membran wenigstens eine Gasaustauschkammer (3b) angeschlossen ist, die aktiv von einem für den CO₂- und/oder O₂-Gasaustausch vorgesehenen Fluid durchströmbar ist, wobei durch die Größe des Volumenstromes des zum Gasaustausch vorgesehenen Fluids die sich beim Atmen asymptotisch einstellende Größe des Endwertes des CO₂- und/oder O₂-Partialdruckes des Atemgases in der Vorrichtung beeinflussbar ist.

2. Vorrichtung nach Anspruch 1, **dadurch gekennzeichnet, dass** der Behälter (2) zur Umgebungsluft hin geschlossen ist.

3. Vorrichtung nach Anspruch 2, **dadurch gekennzeichnet, dass** ein Atemzugbehältnis (7) einen Teil des Volumens (2, 3a, 7) bildet, insbesondere welches direkt an den einstellbaren Behälter (2) angrenzt, wobei das Atemzugbehältnis (7) bewegliche und/oder flexible Wandbereiche (7a) aufweist, so dass das Volumen des Atemzugbehältnisses (7) durch ein- und ausströmendes Atemgas vergrößerbar und verkleinerbar ist.

4. Vorrichtung nach Anspruch 3, **dadurch gekennzeichnet, dass** die Bewegungsweite und damit das maximale Volumen des Atemzugbehältnisses (7) begrenzt ist, insbesondere variabel begrenzbar ist durch wenigstens ein verstellbares Anschlagelement (11), an welches die beweglichen und/oder flexiblen Wandbereiche (7b) des Atemzugbehältnisses (7) bei maximaler Füllung mit Atemgas anlegbar sind.

5. Vorrichtung nach einem der Ansprüche 2 bis 4, **dadurch gekennzeichnet, dass** am Volumen (2, 3a, 7), insbesondere am einstellbaren Behälter (2) ein Ventil (V1) angeordnet ist, durch welches bei einem Überdruck gegenüber der äußeren Umgebung Atemgas aus dem Volumen zur Umgebung hin ablassbar ist und/oder ein Ventil (V2) angeordnet ist, durch welches bei einem Unterdruck gegenüber der äußeren Umgebung Frischluft in das Volumen einsaugbar ist.

6. Vorrichtung nach einem der Ansprüche 2 bis 5, **dadurch gekennzeichnet, dass** am Volumen (2, 3a, 7), insbesondere am einstellbaren Behälter (2) ein Einwege-Ventil angeordnet ist, durch welches beim Einatmen Gas, insbesondere Luft aus dem Behälter und/oder aus dem Atemzugbehältnis (7) über einen Bypass (6) unter Umgehung des an die Gasautauschkammer (3b) angrenzenden Volumenbereichs zur Mündungsöffnung (1) leitbar ist.

7. Vorrichtung nach einem der vorherigen Ansprüche, **dadurch gekennzeichnet, dass** der Behälter (2) zur Umgebungsluft hin offen ist.

8. Vorrichtung nach einem der vorherigen Ansprüche, **dadurch gekennzeichnet, dass** das Volumen (2, 3a, 7) an die jeweils äußere Oberfläche einer Vielzahl gaspermeabler Hohlfasern angrenzt, deren jeweiliges Inneres, insbesondere aktiv, von einem für den Gasaustausch, insbesondere CO₂- und/oder O₂-Gasaustausch vorgesehenen Fluid durchströmbar ist.

9. Vorrichtung nach Anspruch 8, **dadurch gekennzeichnet, dass** die Vielzahl gaspermeabler Hohlfasern gebildet ist durch ein modular bestückbares Aufnahmeelement, in welches wenigstens ein Modul mit einer Vielzahl von Hohlfasern einsetzbar ist, bevorzugt in welches wenigstens zwei Module mit je einer Vielzahl von Hohlfasern einsetzbar ist.

10. Vorrichtung nach einem der vorherigen Ansprüche, **dadurch gekennzeichnet, dass** sie einen Antrieb (9) umfasst, mit dem eine Durchströmung von Fluid durch die wenigstens eine Kammer (3b), insbesondere das jeweilige Innere der Hohlfasern erzeugbar ist, insbesondere wobei der Antrieb (9) ein elektromotorischer Antrieb (9) ist oder durch die Atemgasströmung von ein- und ausgeatmetem Atemgas betrieben ist.

11. Vorrichtung nach einem der vorherigen Ansprüche, **dadurch gekennzeichnet, dass** der Behälter (2) als längenveränderbarer Balg (2) ausgebildet ist oder wenigstens zwei Behälterteile umfasst, die gegeneinander teleskopierbar sind.

12. Verfahren zur Durchführung eines Höhentrainings,wobei eine Person durch eine Mündungsöffnung (1) in eine Trainingsvorrichtung ausatmet und aus dieser Trainingsvorrichtung einatmet, insbesondere unter Nutzung einer Vorrichtung nach einem der vorherigen Ansprüche, **dadurch gekennzeichnet, dass** die sich beim Atmen asymptotisch einstellende Größe des Endwertes des O₂- und/oder CO₂-Partialdrucks in dem aus der Vorrichtung eingeatmeten Atemgas beeinflusst wird durch Austausch von O₂ und/oder CO₂ zwischen dem Atemgas in der Trainingsvorrichtung und einem zum Gasaustausch vorgesehenen Fluid durch wenigstens eine gaspermeable Membran (4) hindurch in Abhängigkeit des Volumenstromes des zum CO₂- und/oder O₂-Gasaustausch vorgesehenen Fluids, das in einer an die gaspermeable Membran (4) angrenzende Gasaustauschkammer (3b) strömt.

13. Verfahren nach Anspruch 12, **dadurch gekennzeichnet, dass** sich mit der Anzahl der Atmungen der O₂- und/oder CO₂- Partialdruck im Volumen der Trainingsvorrichtung asymptotisch an einen Endwert annähert, wobei die Größe des Endwertes eingestellt wird durch die Größe des Volumenstromes des zum Gasaustausch vorgesehenen Fluids durch eine Gasaustauschkammer (3b) hindurch, welche die Membran (4) umfasst.

14. Verfahren nach Anspruch 13 unter Verwendung einer Vorrichtung nach einem der Ansprüche 6 bis 10, **dadurch gekennzeichnet, dass** die Anzahl der Atmungen bzw. die Zeit, nach welcher der Endwert erreicht wird durch das Volumen des Behälters (2) der Trainingsvorrichtung eingestellt wird.

## Claims

1. Apparatus for carrying out altitude training comprising an opening (1), through which respiratory gas can be breathed out by a person into a volume (2, 3a, 7) or can be breathed in from the volume (2, 3a, 7), the said volume (2, 3a, 7) being formed at least partially by a container (2) which is connected to the opening and has an adjustable container volume, and the volume (2, 3a, 7) adjoining one side of at least one gas-permeable diaphragm (4), **characterized in that** at least one gas exchange chamber (3b) is connected on the other side of the at least one diaphragm, which gas exchange chamber (3b) can be flowed through actively by a fluid which is provided for the CO₂ and/or O₂ gas exchange, it being possible for the magnitude of the end value of the CO₂ and/or O₂ partial pressure of the respiratory gas in the apparatus, which magnitude is set asymptotically during breathing, to be influenced by the magnitude of the volumetric flow of the fluid which is provided for the gas exchange.

2. Apparatus according to Claim 1, **characterized in that** the container (2) is closed in the direction of the ambient air.

3. Apparatus according to Claim 2, **characterized in that** a breath vessel (7) forms a part of the volume (2, 3a, 7), which breath vessel (7), in particular, adjoins the adjustable container (2) directly, the breath vessel (7) having movable and/or flexible wall regions (7a), with the result that the volume of the breath vessel (7) can be increased and reduced by way of respiratory gas which flows in and out.

4. Apparatus according to Claim 3, **characterized in that** the range of movement and therefore the maximum volume of the breath vessel (7) are limited, in particular can be limited in a variable manner by way of at least one adjustable stop element (11), against which the movable and/or flexible wall regions (7b) of the breath vessel (7) can bear in the case of maximum filling with respiratory gas.

5. Apparatus according to one of Claims 2 to 4, **characterized in that** a valve (V1) is arranged on the volume (2, 3a, 7), in particular on the adjustable container (2), by way of which valve (V1) respiratory gas can be discharged from the volume towards the surroundings in the case of a positive pressure in comparison with the external surroundings, and/or a valve (V2) is arranged on the said volume (2, 3a, 7), in particular on the said adjustable container (2), by way of which valve (V2) fresh air can be sucked into the volume in the case of a negative pressure in comparison with the external surroundings.

6. Apparatus according to one of Claims 2 to 5, **characterized in that** a one-way valve is arranged on the volume (2, 3a, 7), in particular on the adjustable container (2), by way of which one-way valve, during breathing in, gas, in particular air can be conducted from the container and/or from the breath vessel (7) to the opening (1) via a bypass (6), bypassing the volume region which adjoins the gas exchange chamber (3b).

7. Apparatus according to one of the preceding claims, **characterized in that** the container (2) is open in the direction of the ambient air.

8. Apparatus according to one of the preceding claims, **characterized in that** the volume (2, 3a, 7) adjoins the respective outer surface of a multiplicity of gas-permeable hollow fibres, the respective interior of which can be flowed through, in particular actively, by a fluid which is provided for the gas exchange, in particular the CO₂ and/or O₂ gas exchange.

9. Apparatus according to Claim 8, **characterized in that** the multiplicity of gas-permeable hollow fibres are formed by way of a receiving element which can be equipped in a modular manner and into which at least one module with a multiplicity of hollow fibres can be inserted, preferably into which at least two modules with in each case a multiplicity of hollow fibres can be inserted.

10. Apparatus according to one of the preceding claims, **characterized in that** it comprises a drive (9), by way of a which a throughflow of fluid through the at least one chamber (3b), in particular the respective interior of the hollow fibres, can be generated, the drive (9) being, in particular, an electric motor drive (9) or being operated by way of the respiratory gas flow of respiratory gas which is breathed in and out.

11. Apparatus according to one of the preceding claims, **characterized in that** the container (2) is configured as a variable-length bellows (2) or comprises at least two container parts which can be telescoped with respect to one another.

12. Method for carrying out altitude training, a person breathing out through an opening (1) into a training apparatus and breathing in from the said training apparatus, in particular with the utilization of an apparatus according to one of the preceding claims, **characterized in that** the magnitude of the end value of the O₂ and/or CO₂ partial pressure in the respiratory gas which is breathed in from the apparatus, which magnitude is set asymptotically during breathing, is influenced by the exchange of O₂ and/or CO₂ between the respiratory gas in the training apparatus and a fluid which is provided for the gas exchange through at least one gas-permeable diaphragm (4) in a manner which is dependent on the volumetric flow of the fluid which is provided for the CO₂ and/or O₂ gas exchange and flows in a gas exchange chamber (3b) which adjoins the gas-permeable diaphragm (4).

13. Method according to Claim 12, **characterized in that** the O₂ and/or CO₂ partial pressure in the volume of the training apparatus approaches an end value asymptotically with the number of respirations, the magnitude of the end value being set by way of the magnitude of the volumetric flow of the fluid which is provided for the gas exchange through a gas exchange chamber (3b) which comprises the diaphragm (4).

14. Method according to Claim 13 with the use of an apparatus according to one of Claims 6 to 10, **characterized in that** the number of respirations or the time, after which the end value is reached, is set by way of the volume of the container (2) of the training apparatus.

## Revendications

1. Dispositif permettant de mettre en oeuvre un entraînement à l'altitude, comprenant un orifice buccal (1), à travers lequel un gaz respiratoire peut être expiré par une personne dans un volume (2, 3a, 7) ou inspiré à partir du volume (2, 3a, 7), dans lequel ce volume (2, 3a, 7) est formé au moins en partie par un récipient (2) de capacité réglable et raccordé à l'orifice buccal et dans lequel le volume (2, 3a, 7) est adjacent à un côté d'au moins une membrane (4) perméable au gaz, **caractérisé en ce qu'**à l'autre côté de ladite au moins une membrane (4) est raccordée une chambre d'échange de gaz (3b), qui peut être parcourue de façon active par un fluide prévu pour l'échange de gaz CO₂ et/ou O₂, dans lequel la grandeur se réglant de façon asymptotique lors de la respiration de la valeur finale de la pression partielle de CO₂ et/ou d'O₂ du gaz respiratoire peut être influencée dans le dispositif par la grandeur du flux de volume du fluide prévu pour l'échange de gaz.

2. Dispositif selon la revendication 1, **caractérisé en ce que** le récipient (2) est fermé à l'égard de l'air ambiant.

3. Dispositif selon la revendication 2, **caractérisé en ce qu'**un ballon respiratoire (7) forme une partie du volume (2, 3a, 7), en particulier qui est directement adjacent au récipient réglable (2), dans lequel le ballon respiratoire (7) présente des régions de paroi mobiles et/ou flexibles (7a), de telle manière que le volume du ballon respiratoire (7) puisse être augmenté ou diminué par le gaz respiratoire expiré ou inspiré.

4. Dispositif selon la revendication 3, **caractérisé en ce que** l'ampleur du mouvement et dès lors le volume maximal du ballon respiratoire (7) est limité(e), en particulier peut être limité(e) de façon variable par au moins un élément de butée déplaçable (11), sur lequel les régions de paroi mobiles et/ou flexibles (7b) du ballon respiratoire (7) peuvent être appliquées en cas de remplissage maximal avec du gaz respiratoire.

5. Dispositif selon l'une quelconque des revendications 2 à 4, **caractérisé en ce qu'**une soupape (V1) est disposée sur le volume (2, 3a, 7), en particulier sur le récipient réglable (2), par laquelle du gaz respiratoire peut être expulsé hors du volume vers l'atmosphère en cas de surpression par rapport à l'atmosphère extérieure et/ou une soupape (V2) y est disposée, par laquelle de l'air frais peut être aspiré dans le volume en cas de dépression par rapport à l'atmosphère extérieure.

6. Dispositif selon l'une quelconque des revendications 2 à 5, **caractérisé en ce qu'**une soupape à une voie est disposée sur le volume (2, 3a, 7), en particulier sur le récipient réglable (2), par laquelle lors de l'inspiration du gaz, en particulier de l'air, peut être conduit du récipient et/ou du ballon respiratoire (7) à l'orifice buccal (1) via une dérivation (6) avec contournement de la région de volume adjacente à la chambre d'échange de gaz (3b).

7. Dispositif selon l'une quelconque des revendications précédentes, **caractérisé en ce que** le récipient (2) est ouvert en direction de l'air ambiant.

8. Dispositif selon l'une quelconque des revendications précédentes, **caractérisé en ce que** le volume (2, 3a, 7) est adjacent à la surface respectivement extérieure d'une multiplicité de fibres creuses perméables au gaz, dont l'intérieur respectif peut être parcouru, en particulier de façon active, par un fluide prévu pour l'échange de gaz, en particulier pour l'échange de gaz CO₂ et/ou O₂.

9. Dispositif selon la revendication 8, **caractérisé en ce que** la multiplicité de fibres creuses perméables au gaz est formée par un élément de réception à garnir de façon modulaire, dans lequel au moins un module avec une multiplicité de fibres creuses peut être inséré, de préférence dans lequel au moins deux modules avec chacun une multiplicité de fibres creuses peuvent être insérés.

10. Dispositif selon l'une quelconque des revendications précédentes, **caractérisé en ce qu'**il comprend un entraînement (9), avec lequel on peut produire un écoulement de fluide à travers ladite au moins une chambre (3b), en particulier l'intérieur respectif des fibres creuses, en particulier dans lequel l'entraînement (9) est un entraînement par moteur électrique (9) ou est effectué par le courant de gaz respiratoire de gaz respiratoire inspiré et expiré.

11. Dispositif selon l'une quelconque des revendications précédentes, **caractérisé en ce que** le récipient (2) est réalisé sous la forme d'un soufflet de longueur variable (2) ou comprend au moins deux parties de récipient, qui sont télescopiques l'une par rapport à l'autre.

12. Procédé permettant de mettre en oeuvre un procédé d'entraînement à l'altitude, dans lequel, par un orifice buccal (1), une personne expire de l'air dans un dispositif d'entraînement et inspire de l'air de ce dispositif d'entraînement, en particulier en utilisant un dispositif selon l'une quelconque des revendications précédentes, **caractérisé en ce que** l'on influence la grandeur se réglant de façon asymptotique lors de la respiration de la valeur finale de la pression partielle d'O₂ et/ou de CO₂ dans le gaz respiratoire inspiré à partir du dispositif par échange d'O₂ et/ou de CO₂ entre le gaz respiratoire dans le dispositif d'entraînement et un fluide prévu pour l'échange de gaz à travers au moins une membrane perméable au gaz (4) en fonction du flux de volume du fluide prévu pour l'échange de gaz CO₂ et/ou O₂, qui s'écoule dans une chambre d'échange de gaz (3b) adjacente à la membrane perméable au gaz (4).

13. Procédé selon la revendication 12, **caractérisé en ce que** la pression partielle d'O₂ et/ou de CO₂ dans le volume du dispositif d'entraînement s'approche de façon asymptotique d'une valeur finale avec le nombre des respirations, dans lequel on règle la grandeur de la valeur finale par la grandeur du flux de volume du fluide prévu pour l'échange de gaz à travers une chambre d'échange de gaz (3b), qui comprend la membrane (4).

14. Procédé selon la revendication 13 avec utilisation d'un dispositif selon l'une quelconque des revendications 6 à 10, **caractérisé en ce que** l'on règle le nombre des respirations ou le temps après lequel la valeur finale est atteinte par le volume du récipient (2) du dispositif d'entraînement.
